# NEW EUROPEAN PATENT SPECIFICATION

(11) **EP 2 064 279 B2**
(45) Date of publication and mention of the opposition decision: **18.07.2018**
(45) Mention of the grant of the patent: 07.09.2011
(21) Application number: 08773962.9
(22) Date of filing: 10.07.2008
(51) Int. Cl.: C08K 5/00, C07C 409/08

(54) **POLYMER COMPOSITION COMPRISING AN ORGANIC PEROXIDE FREE RADICAL GENERATING AGENT, PREPARATION OF THE COMPOSITION, CROSSLINKABLE CABLE PRODUCED USING THE COMPOSITION AND ORGANIC PEROXIDES**
POLYMERZUSAMMENSETZUNG, UMFASSEND EIN AUF ORGANISCHES PEROXID BASIERTES, FREIE RADIKALE ERZEUGENDES MITTEL, HERSTELLUNG DER ZUSAMMENSETZUNG, UNTER VERWENDUNG DER ZUSAMMENSETZUNG HERGESTELLTES VERNETZBARES KABEL UND ORGANISCHE PEROXIDE
COMPOSITION DE POLYMÈRE COMPRENANT UN AGENT GÉNÉRATEUR DE RADICAUX LIBRES DE PEROXYDE ORGANIQUE, PRÉPARATION DE LA COMPOSITION, CÂBLE RÉTICULABLE PRODUIT EN UTILISANT LA COMPOSITION, ET PEROXYDES ORGANIQUES

(30) Priority: 12.07.2007 EP 07112305
(43) Date of publication of application: 03.06.2009
(73) Proprietor: Borealis Technology OY, 06101 Porvoo (FI)
(72) Inventor: SMEDBERG, Annika, S-471 61 Myggenäs (SE); GUSTAFSSON, Bill, S-444 41 Stenungsund (SE); NILSSON, Daniel, S-415 12 Göteborg (SE)
(74) Representative: Dehns
(86) International application number: PCT/EP2008/005640
(87) International publication number: WO 2009/007117

(56) References cited:
- EP-A- 0 370 551
- EP-A- 0 943 650
- EP-A- 0 970 975
- EP-A- 1 925 466
- EP-B1- 2 064 280
- WO-A-01/66628
- WO-A-02/44265
- WO-A-91/16716
- WO-A-99/23164
- WO-A-99/27015
- WO-A1-96/03444
- DD-A- 63 491
- GB-A- 1 131 825
- GB-A- 1 294 154
- GB-A- 1 588 870
- JP-A- H 036 246
- US-A- 2 528 523
- US-A- 2 601 224
- US-A- 3 079 370
- US-A- 3 578 647
- US-A- 3 738 866
- US-A- 5 153 272
- MILAS N. ETAL: 'The synthesis and thermal decomposition of Di-triethylmethyl, t-butyl pentamethylethyl and t-butyl 1-methylcyclohexyl-1 peroxides 1' JOURNAL OF THE AMERICAN CHEMICAL SOCIETY vol. 68, no. 10, 10 October 1946, pages 1938 - 1940
- KOCHI J.K.: 'Chemistry of alkoxy radicals: Cleavage reactions' JOURNAL OF THE AMERICAN CHEMICAL SOCIETY vol. 84, no. 7, 05 April 1962, pages 1193 - 1197

## Description

### Field of invention

The invention relates to a process for modifying a polymer composition, to modified polymer compositions, to an article, preferably wire or cable, comprising said modified polymer composition, to a process for preparing an article, preferably a wire or cable, to the use of said modified polymer in one or more layers of a wire or cable, as well as to a compound for use as a radical generating agent for modifying a polymer composition.

### Background art

It is known to use free radical generating agents for modifying a product, such as a polymer composition via a radical reaction.

Free radical agents are used e.g. to initiate (a) crosslinking in a polymer, i.a. primarily formation of interpolymer crosslinks (bridges) by radical reaction, (b) grafting in a polymer, i.e. introduction of compounds to a polymer chain (to backbone and/or side chains) by radical reaction, and (c) visbreaking in a polymer, i.e. modification of melt flow rate (MFR) of a polymer by radical reaction. These polymer modifications are well known in the art.

Many peroxides are known. EP-A-1925466 describes butyl rubber which contains organoperoxide for use in the manufacture of tyres. The use of dicumylperoxide is preferred.

In WO02/44265, the peroxide used in these examples is Varox DBPH which is 2,5 dimethyl 2,5(di(tbutylperoxide) hexane.

WO01/66628 describes compositions containing a polymer and a free radical generating agent all of which have two methyl groups adjacent to the peroxide linkage.

EP-A-0970975 describes the peroxide DBPH, i.e. 2,5-bis(tert-butylperoxy)-2,5-dimethylhexane. EP-A-943650 also exemplifies peroxides such as DBHP.

The free radical generating agents in WO99/27015 are ones which have hitherto been used conventionally in the art. Dicumylperoxide is used in the examples.

WO99/23164 discloses a long list of specific peroxides on page 6 concluding that dibenzoyl peroxide and di(4-tbutylcyclohexyl)peroxycarbonate are preferred.

It is only in the examples of WO91/16716 that specific peroxides are listed but again Lupersol 101 peroxide is not within the scope of this application.

EP-A-0370551 contains a long list of peroxides but exemplifies 1,4-di(2-tert-butylperoxyisopropyl)benzene which has two methyl groups adjacent the peroxide linkage.

The list of peroxides (with two methyls at the carbon atoms adjacent to O-O) in column 5 of US3578647 covers peroxides which we exclude.

When added to a polymer composition, free radical generating agents act by generating radicals, typically by decomposing to radicals, under conditions which enable the radical formation. The decomposed radicals initiate further radical reactions within a polymer composition. The resulting decomposition products of the free radical generating agent are typically a result of several reactions of the decomposition products of initial radical forming reaction. Said resulting decomposition products typically remain in the modified polymer and may include detrimental, undesired decomposition products.

Peroxides are very common free radical generating agents used i.a. in the polymer industry for said polymer modifications. The resulting decomposition products of peroxides may include volatile by-products. For example, dicumylperoxide, which is commonly used peroxide in polymer field, decomposes i.a. to methane, acetophenone and cumylalcohol during the radical formation step, e.g. during a crosslinking step. The formed gaseous methane (CH₄) is flammable, explosive and volatile and thus a risk in a working environment.

In wire and cable applications, a typical cable comprises at least one conductor surrounded by one or more layers of polymeric materials. In some power cables, including medium voltage (MV), high voltage (HV) and extra high voltage (EHV) cables, said conductor is surrounded by several layers including an inner semiconductive layer, an insulation layer and an outer semiconductive layer, in that order. The cables are commonly produced by extruding the layers on a conductor. One or more of said layers are then typically crosslinked to improve i.a. deformation resistance at elevated temperatures, as well as mechanical strength and/or chemical resistance, of the layer(s) of the cable. The free radical generating agent, such as peroxide, is typically incorporated into the layer material prior to the extrusion of the layer(s) on a conductor. After formation of the layered cable, the cable is then subjected to a crosslinking step to initiate the radical formation and thereby crosslinking reaction.

The decomposition products of the free radical forming agent remain mostly captured within the cable layer after crosslinking. This causes problems in view of the cable manufacturing process as well as in view of the quality of the final cable.

Accordingly, after crosslinking the cable must be cooled with great care to prevent the gaseous volatile decomposition products like methane forming voids within the polymer layer. These voids have typically an average diameter of between 10 to 100 µm. Partial discharges can take place in such voids within a cable that is subjected to an electrical field and thereby reduce the electrical strength of the cable.

The MV, HV and EHV power cables must have high layer quality in terms of safety during installation and in end use thereof. In service, volatile decomposition products in a cable resulting from a crosslinking step can create a gas pressure and thus cause defects in the shielding and in the joints. E.g. when a cable is equipped with a metal barrier, then the gaseous products can exert a pressure, especially on the joints and terminations, whereby a system failure may occur.

For the above reasons the volatile decomposition products, such as methane e.g. where dicumylperoxide is used, are conventionally reduced to a minimum or removed after crosslinking and cooling step. Such a removal step is generally known as a degassing step.

The degassing step is time and energy consuming and is thus a costly operation in a cable manufacturing process. Degassing requires large heated chambers which must be well ventilated to avoid the build-up of e.g. flammable methane and ethane. The cable, typically wound to cable drums, is normally degassed at elevated temperature in the range of 50-80°C, e.g. 60-70°C, for lengthy time periods. At these temperatures however, thermal expansion and softening of the insulation can occur and lead to undue deformation of the formed cable layers resulting directly in failures of the cable. The degassing of MV, HV and EHV cables with high cable weight needs thus often be carried out at decreased temperatures.

Accordingly, there is a need to find new solutions to overcome the prior art problems.

### Objects of the invention

One of the objects of the present invention is to provide an alternative process for modifying a polymer composition by using a free radical generating agent with superior properties.

Another object of the invention is to provide a cross-linkable cable produced from said modified polymer composition, which comprises one or more layers comprising said modified polymer composition, which cable has highly advantageous properties, such as high quality and superior processability properties.

A further object of the invention is to provide a process for crosslinking a cable using said modified polymer composition, preferably a cable, as defined above, which process enables the preparation of high quality products with shorter production time and/or lower energy consumption.

A further object of the invention is to provide a process for using said modified polymer composition, preferably a cable, as defined above, which process enables the preparation of high quality products with shorter production time and/or lower energy consumption.

The invention and further objects thereof are described and defined in details below.

### Description of the invention

The objects of the invention are solved by the compounds, polymer compositions, end products and processes as defined i.a. in the description and claims below.

Accordingly, in a first embodiment, the invention provides:
a polymer composition comprising
   - at least one polymer component, e.g. in a form of (1) polymer powder, (2) polymer pellets or (3) a polymer melt. whereby said (1) polymer powder or, preferably, (2) polymer pellets are optionally contained in a container; and
   - at least one free radical generating agent
wherein the at least one free radical generating agent is a compound which is an organic peroxide of formula (I) 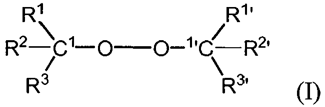
   wherein R¹ and R^{1,} are the same and each represents methyl; and
   R² and R³ together with C¹ atom to which they are attached form a saturated or partially unsaturated mono- or bicyclic (C4-C14)carbocyclic ring,
   and R^{2,} and R^{3,} together with the carbon atom (C¹') to which they are attached form a saturated or partially unsaturated mono- or bicyclic (C4-C14)carbocyclic ring whereby the ring system formed by R² and R³ together with C¹ is preferably identical to a ring system formed by R^{2,} and R^{3,} together with C¹'; or
   R¹ and R^{1,} are the same and represent an unsubstituted, monocyclic (C5-C7)aryl;
   R² and R^{2,} are the same and are methyl; and
   R³ and R^{3,} are the same and are straight chain (C6-C30)alkyl.

It is preferred if when the compound of formula (I) is used as a free radical generating agent the compound results in methane (CH₄) content of less than 300 ppm (weight), preferably of less than 200 ppm (weight), preferably of less than 100 ppm (weight) more preferably of 0 to 50 ppm (weight), as a decomposition product thereof.

Generally, in above and below definitions the given values in ppm for methane and/or other volatile content are determined by gas chromatography from the obtained crosslinked polymer composition as such or from a crosslinked cable layer, depending on the definition, according to a method as described below under "GC-analysis protocol". Accordingly, the produced methane or other volatile content can equally be determined from a crosslinked polymer composition as such or from a crosslinked manufactured article thereof, as desired, each consisting of the polymer composition of the invention. The sample under the test is crosslinked using the test free radical generating agent in such an amount which results in a crosslinking degree expressed as gel content of 50 %, and preferably gel content of at least 50 %. The gel content (%) is measured according to ASTM D2765-01 Method A or B (depending on the nature of the sample). Such a crosslinked sample is then used for preparing the sample for volatile content measurement of GC-analysis protocol. Without limiting to any theory, the terms "a decomposition product(s) thereof" or "a decomposition product of a free radical generating step" etc. as used above and below mean herein a by-product(s) formed during a free radical generating step, e.g. crosslinking step, and possibly also during the cooling step, by initiation of the free radical generating agent, as well known in the art. As an example methane may be one decomposition product which is an undesired decomposition product of the invention. Further decomposition products are specified below, which may not be desired in various embodiments of the invention.

In a preferred embodiment, the compound of formula (I) is without CH₄ as a decomposition product thereof.

The term "without resulting in CH₄ as a decomposition product thereof' means that a compound of formula (I) generates no methane, or in alternative terms does not decompose to the undesired volatile CH₄ by-product during a radical formation step in an industrial process.

The solution of the invention provides a new principal which is surprising and unobvious, since in the prior art there is no teaching or any indication to modify the free radical generating agent in order to avoid formation of CH₄ as a decomposing product during the free radical formation step in an industrial process. For example, in crosslinking applications, the prior art has proposed merely solutions relating to balance the amount of free radical generating agent and the needed degree of crosslinking.

In one embodiment said compound of formula (I) results in reduced amount of or preferably does not decompose to low molecular weight compounds selected from (C1-C3)alkanes when generating free radicals, e.g. in industrial applications.

In another embodiment of the invention it is advantageous that said compound of formula (I) as a free radical generating agent results in reduced amount of or preferably is without (C1-C4)alkanes as decomposition products thereof when generating free radicals, e.g. in industrial applications.

In embodiments, wherein very high quality is required for the products modified by using a free radical agent, then it is preferable that said compound of formula (I) results in reduced amount of or is preferably without (C1-C6)alkanes as decomposition products thereof during a free radical forming step, e.g. in an industrial process.

It is preferred if the compound of formula (I) for use as a free radical generating agent does not result in (i.e. is without) decomposition products, preferably hydrocarbon decomposition products, having a boiling point at atmospheric pressure of less than 50°C, preferably less than 80°C, or in some embodiments even less than 100°C may be desired. "Hydrocarbon" has the same meaning as given below for "hydrocarbyl" which represents a hydrocarbon as a monovalent substituent.

US3079370 discloses a generic group of peroxides, but without specifying any peroxides with less than two methyls at carbon atoms corresponding C¹ and C¹' in formula (I) above.

It will be appreciated that whist some peroxides are known per se they have not been disclosed in connection with polymer modification, in particular specific types of polymer modification and especially in cable cross-linking.

The terms used for defining the compounds of formula (I) are well known in the organic chemistry. E.g. in moieties defined in formula (I):

The term "unsubstituted" naturally means that no substituent is present.

The following subgroups of the compound of formula (I) represent some preferable embodiments and variants of the invention. It is also understood that said below subgroups further specify the substituents given above in formula (I). Each subgroups definition can be combined with any other subgroup to define further preferred subgroups within the broadest scope of compounds of formula (I).

Moreover said, general definition for compounds of formula (I), thereof, can be combined in any combination in their uses for modifying polymers, to modification methods, to modified polymers and to articles comprising said modified polymers, as well as to preparation process thereof, which all aspects of the invention are discussed below.

In a preferred embodiment of the invention compounds of formula (I) are symmetrical.

In a subgroup (3) of the compound of formula (I) as defined above, R² and R³ together with the carbon atom (C¹) to which they are attached form an saturated or partially unsaturated mono- or bicyclic (C4-C14) carbocyclic ring, preferably unsubstituted saturated monocyclic (C5-C8) carbocyclic ring, such as cyclopentyl, cyclohexyl or cycloheptyl, preferably cyclohexyl or cyclopentyl.

In a subgroup (4) of the compound of formula (I) as defined above, R^{2,} and R^{3,} together with the carbon atom (C¹') to which they are attached form a saturated or partially unsaturated mono- or bicyclic (C4-C14) carbocyclic ring, preferably unsubstituted saturated monocyclic (C5-C8) carbocyclic ring, such as cyclopentyl, cyclohexyl or cylcoheptyl, preferably cyclohexyl or cyclopentyl.

In an even preferable subgroup (5b) of the compound of formula (I) as defined above, R² and R³ together with the carbon atom (C¹) to which they are attached form a ring system as defined in subgroup (3) and R^{2,} and R^{3,} together with the carbon atom (C¹') to which they are attached form a ring system as defined in subgroup (4), whereby the ring system formed by R^{2,} and R^{3,} together with the carbon atom (C¹') is identical to the ring system formed by R² and R³ together with the carbon atom (C¹).

One of the preferred compounds is di-(1-methylcyclohexyl) peroxide (formula Ia):

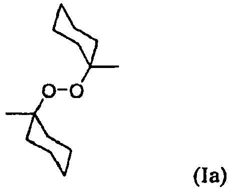

Another preferred compound is (Ia'), di(I-methylcyclopentyl) peroxide.

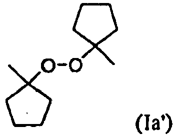

In preferable subgroup (Ib) of compounds of formula (I) as defined above, R¹ and R^{1,} are both same and represent an unsubstituted, monocyclic (C5-C7)aryl;
R² and R^{2,} are same and are both methyl; and
R³ and R^{3,} are same and are unsubstituted straight chain (C6-C30)alkyl, such as (C6-C20)alkyl.

One of the preferred compounds of formula (I) of this preferable subgroup of (Ib) is Di-(1-methyl-1-phenylundecyl) peroxide (formula Ib):

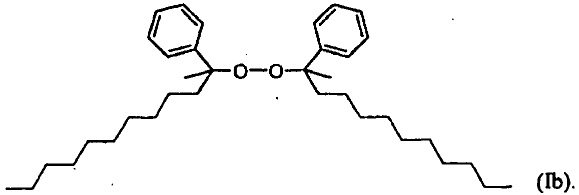

Other preferred compounds of subgroup (Ib) include di-(1-methyl-1-phenylheptyl) peroxide

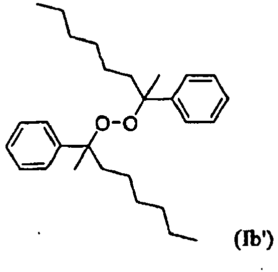

In other preferred embodiments of the invention none of R¹-R³ or R^{1,}-R^{3,} represents an aromatic group.

Where one or more of R¹-R³ or R^{1,}-R^{3,} represents an aromatic radical this is especially preferably a phenyl group.

Where one or more of R¹-R³ or R^{1,}-R^{3,} represents a cycloalkyl radical this is especially preferably a cyclohexyl or cyclopentyl group.

In the compounds of formula (I) there are preferably no more than two cycloalkyl groups. In further preferred compounds there are no more than two cyclic groups in the compound of formula (I). In a most preferred embodiment of the invention there are two cyclic groups which each are formed by R² and R³ together with C¹ and by R^{2,} and R^{3,} together with C¹.

These preferred embodiments apply to any compound of formula (I), in particular any compounds forming part of the sub groups above.

Highly preferred compounds of the invention are therefore of formula (II) 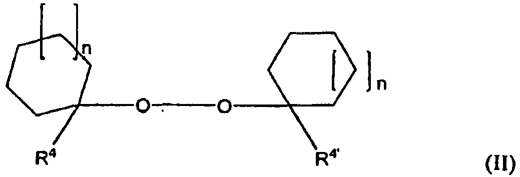 wherein n 0 to 3, preferably 0 or 1 forming a cyclopentyl or cyclohexyl group respectively, R⁴ and R^{4,} each independently represent methyl.

Further highly preferred compounds of the invention are also of formula (III) 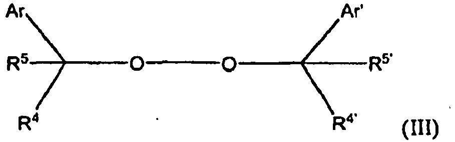 wherein Ar and Ar' independently represent a phenyl, benzyl or naphthyl group
R⁴ and R^{4,} each are methyl; and
R⁵ and R^{5,} each independently represent a straight chain alkyl group having C6-30 carbon atoms, preferably 6 to 20, more preferably 6 to 12 carbon atoms,

Most preferred is compounds of formula (II) as defined above.

### Preparation of the compounds of formula (I)

The compounds of formula (I) include novel and known compounds. The use of the known compounds as a free radical generating agent, preferably for modifying a polymer composition, is novel. Thus said known compounds may be commercially available. Alternatively, the compounds can be prepared according to or analogously to known methods described in the chemical literature.

As an example, the compounds (I) as defined above can be prepared according to the following scheme 1 using known procedures which are described in a literature and well known for a skilled person in the art.

Peroxides of formula (I) as defined above can be prepared in several known ways, and more specifically tertiary peroxides can be prepared from the corresponding tertiary alcohols under acidic conditions to give compounds (I). The alcohols are either commercially available, or can be prepared from a suitable ketone combined with a organometallic reagent, more specifically a Grignard (RMgX, where X is an halogen) or organolithium (RLi) reagent.

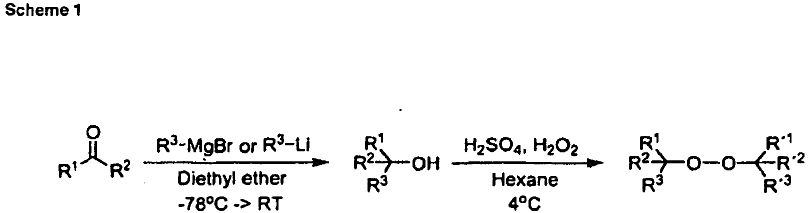

References to synthetic methods are as follows:
1) Milas, N.A., Surgenor, D.M., J. Am. Chem. Soc., 643-644, 1946
2) Hey, D.H., Stirling, C.J.M., Williams, G.H, J. Chem. Soc., 1054-1058, 1957
3) Organic Synthesis, Smith, M.B., The McGraw-Hill Companies Inc., 2002

Work up procedures are routine. The formed tertiary alcohol and corresponding peroxide can be purified by removing the solvent *in vacuo* and purifying the residue by any of the methods known to those skilled in the art, such as crystallization.

Compounds of formula (I) can also be prepared from tertiary alcohols via conversion to a hydroperoxide -OOH type compound. This process allows the preparation of asymmetrical peroxides. Thus for example, a tertiary alcohol can be converted to a tertiary halide and reacted with hydrogen peroxide, perhaps in the presence of a promoter such as silver trifluoroacetate and a non nucleophilic base such as sodium hydrocarbonate to form a teriary hydroperoxide. The tertiary hydroperoxide can then be reacted with the a tertiary bromide (perhaps the same as used earlier in the reaction or optionally a difference tertiary bromide) to form the final diperoxide materials of formula (I). Again, a promoter such as silver trifluoroacetate/NaHCO₃ might be used. These reactions are summarised in the scheme below:

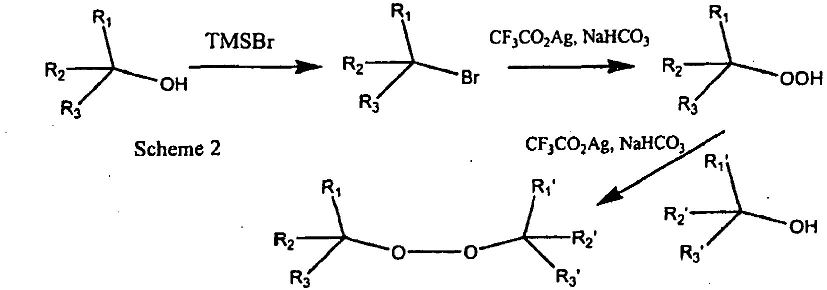

In view of the low levels of volatile decomposition products formed during activation of the peroxides of the invention, the present invention reduces or minimises the fire, explosion and health risks in an working environment caused by the use of free radical generating agents compared to the prior art.

The first group and the second of compounds of the invention as defined above and in claim 1-3 in terms of the decomposition product(s) thereof, the third group of compounds of formula (I) of the invention as defined above with a general formula and by means of the preferable subgroups, in any combinations, as well as in claims 4-15 below, are abbreviated herein below as "Compound of the invention" for the sake of convenience, only. The preferred subgroup of Compound of the invention is compounds of formula (I) including the preferable embodiments and subgroups as defined above and in claims.

### End uses and end applications of the invention

### I. Modification method of polymers

The invention is further directed to the use of Compound of formula (I) as a free radical generating agent for modifying polymers by radical formation. Also a process for modifying polymers by radical reaction using a free radical generating agent is provided, wherein said free radical generating agent is Compound of formula (I).

### 1. Crosslinking of polymers

One preferable embodiment of said modification method of polymers is crosslinking of polymers by radical reaction using one or more free radical generating agents, wherein at least one said free radical generating agent is Compound as defined above.

The term "crosslinking" is well known and commonly used in the polymer field and means forming, primarily, of interpolymer crosslinks (bridges) via radical reaction.

In principle, the polymers usable in the crosslinking process of the present invention are not limited and can be polymers of any type.

In one preferable embodiment, said crosslinkable polymer is a polyolefin which can be a homopolymer of an olefin or a copolymer of an olefin with one or more comonomers.

One preferable group of crosslinkable polyolefins includes homopolymer of ethylene or copolymer of ethylene with one or more comonomers, such as 1) a branched polyethylene homo- or copolymer produced in high pressure by radical polymerisation and well known as low density polyethylene (LDPE) homo or copolymer or 2) a linear polyethylene homo- or copolymer produced by low pressure polymerisation using a coordination catalyst, such as well known linear very low density polyethylene, linear low density polyethylene (LLDPE), medium density polyethylene (MDPE) or high density polyethylene (HDPE), 3) polypropylene polymers, including homopolymers and random polymers of polypropylene and heterophasic copolymer of propylene, or 4) polybutylene polymers, as non-limiting examples only.

One preferable group of crosslinkable ethylene polymers is 1) LDPE homopolymer or LDPE copolymer with one or more comonomers including C3 or higher alphaolefin comonomer(s), polar comonomers and comonomers with at least two double bonds, such as diene comonomers. High pressure polymerisation is a well known technology in the polymer field and can be effected in a tubular or an autoclave reactor, preferably, in a tubular reactor. Further details about high pressure radical polymerisation are given in WO 93/08222.

In one preferable embodiment the crosslinkable LDPE is an LDPE copolymer of ethylene with a polar group containing comonomer(s) and, optionally, with other comonomer(s). As examples of comonomers having polar groups may be mentioned the following: (a) vinyl carboxylate esters, such as vinyl acetate and vinyl pivalate, (b) (meth)acrylates, such as methyl(meth)acrylate, ethyl(meth)acrylate, butyl(meth)acrylate and hydroxyethyl(meth)acrylate, (c) olefinically unsaturated carboxylic acids, such as (meth)acrylic acid, maleic acid and fumaric acid, (d) (meth)acrylic acid derivatives, such as (meth)acrylonitrile and (meth)acrylic amide, and (e) vinyl ethers, such as vinyl methyl ether and vinyl phenyl ether.

Amongst these comonomers, vinyl esters of monocarboxylic acids having 1 to 4 carbon atoms, such as vinyl acetate, and (meth)acrylates of alcohols having 1 to 4 carbon atoms, such as methyl (meth)acrylate, are preferred.

Especially preferred comonomers are butyl acrylate, ethyl acrylate and methyl acrylate. Still more preferably, the polar copolymer comprises a copolymer of ethylene with C₁- to C₄-alkyl acrylate, such as methyl, ethyl, propyl or butyl acrylates or vinyl acetate, or any mixture thereof.

The term "(meth)acrylic acid" and "(meth)acrylate" are intended to embrace both acrylic acid and methacrylic acid and, respectively "methacrylate" and "acrylate".

Another preferable group of crosslinkable polymers are unsaturated polymers, wherein the unsaturation is provided by double bonds, preferably carbon-carbon (C-C) double bonds. More preferably, said unsaturated polymer comprises carbon-carbon double bonds, in a total amount of carbon-carbon double bonds/1000 carbon atoms of 0.1 or more, more preferably of 0.2 or more, and most preferably more than 0.37, more preferably at least 0.40. Preferably in this embodiment the total amount of carbon-carbon double bonds in the unsaturated polymer is of at least 0.6/1000 carbon atoms. The upper limit of the amount of carbon-carbon double bonds present in the unsaturated polymer is not limited and may preferably be less of than 5.0/1000 carbon atoms, preferably less than 3.0/1000 carbon atoms, or more preferably less than 2.5/1000 carbon atoms.

Unsaturated polymer means herein a homopolymer, wherein the unsaturation is provided by a chain transfer agent, or a copolymer, wherein the unsaturation is provided by polymerizing a monomer in the presence of at least a polyunsaturated comonomer and optionally in the presence of a chain transfer agent. Preferably, said carbon-carbon double bonds present in the unsaturated polymer include vinyl groups, which vinyl groups originate preferably from i) a polyunsaturated comonomer, from ii) a chain transfer agent, or from iii) any mixture thereof.

Said polyunsaturated comonomer is preferably a diene, preferably a diene which comprises at least eight carbon atoms, the first carbon-carbon double bond being terminal and the second carbon-carbon double bond being non-conjugated to the first one. Preferred dienes are selected from C₈ to C₁₄ non-conjugated dienes or mixtures thereof, more preferably selected from 1,7-octadiene, 1,9-decadiene, 1,11-dodecadiene, 1,13-tetradecadiene, 7-methyl-1,6-octadiene, 9-methyl-1,8-decadiene, or mixtures thereof. Even more preferably, the diene is selected from 1,7-octadiene, 1,9-decadiene, 1,11-dodecadiene, 1,13-tetradecadiene, or any mixture thereof.

In addition to the vinyl groups originating from the polyunsaturated comonomer, the content of vinyl groups may also be originated, alone or additionally, from a chain transfer agent which introduces vinyl groups, such as propylene.

Preferred unsaturated polyolefins are crosslinkable LDPE as defined above which has the above C-C double bond unsaturation provided by copolymerising ethylene in high pressure together with at least a polyunsaturated comonomer, preferably diene as defined above, and/or in the presence of chain transfer agent as defined above. Moreover, said crosslinkable LDPE suitable for the present invention is preferably an LDPE copolymer which is prepared by copolymerising ethylene with at least one polyunsaturated comonomer, preferably with at least one diene as defined above. Such polymers are well known and described e.g. in WO93/08222, EP 1695996 or WO2006/131266.

Alternatively, or in addition to the double bonds of the unsaturated polymer, the polymer composition of the invention may contain additives, such as known crosslinking boosters, which provide double bonds to the polymer composition. In such case the amount of these double bonds may be the preferable amount as given above for C-C double bonds of unsaturated polymer, or if an unsaturated polymer is present, then the above given preferable amounts of C-C double bonds of polymer is preferably the total sum of the double bonds originating from unsaturated polymer and from the double bonds originating from such additives.

Also linear ethylene polymers prepared using said low pressure polymerisation are very suitable for the crosslinking of the invention. As an example LLDPE, MDPE and HDPE polymers can be mentioned. They can be produced in a known manner in a single or multistage processes using one or more of e.g. Ziegler-Natta catalysts, single site catalysts, including metallocenes and non-metallocenes, and Cr-catalysts. All said catalysts are very well known in the field. The multistage process includes any combinations of polymerisation processes, such as slurry polymerisation, solution polymerisation, gas phase polymerisation, or any combinations thereof, in any order.

Generally, crosslinkable polymers that are usable in the present invention include any known polymers, e.g. commercially available polymers, or they can prepared in a known manner according to or analogously to polymerisation process described in the literature. Naturally any mixtures of polymers can also be used.

The amount of the Compound of formula (I) as a free radical generating agent used for the crosslinking is not critical and can vary depending on the desired crosslinking degree and the type of the crosslinkable polymer. As an example only, the amount of said free radical generating agent may be less than 10.0 wt%, less than 6.0 wt%, less than 5.0 wt%, less than 3.5 wt%, e.g. between 0.1 to 3.0 wt%, such as 0.2 to 2.6 wt%, based on the weight of the crosslinkable polymer composition, depending i.a. on the molecular weight of Compound and the desired degree of crosslinking.

The crosslinking may be carried out in a known manner, typically in elevated temperature, such as 140°C or more, preferably 150°C or more. And said step may be effected under atmospheric or typically slightly pressurised conditions, e.g. up to 20 bar, e.g. up to about 13 bar, pressure.

### 2. Grafting of polymers

Another embodiment of the modification method of the invention is a grafting of a polymer by radical reaction using a free radical generating agent, wherein said free radical generating agent is Compound of formula (I).

The "grafting of a polymer" is a well known modification method and means introducing compounds to the polymer chain (backbone and/or side chain) by radical reaction. Typically the compounds to be incorporated by grafting are unsaturated. Such grafting process are described in the literature, e.g. in US 3,646,155 and US 4,117,195.

In one preferable embodiment of grafting of the invention the polymer is grafted by an unsaturated silane compound represented by the formula R¹⁹SiR²⁰_{q}Y_{3-q} (I) wherein
R¹⁹ is an ethylenically unsaturated hydrocarbyl, hydrocarbyloxy or (meth)acryloxy hydrocarbyl group,
R²⁰ is an aliphatic saturated hydrocarbyl group,
Y which may be the same or different, is a hydrolysable organic group and
q is 0, 1 or 2.

Commonly used compounds are vinyl trimethoxysilane, vinyl bismethoxyethoxysilane, vinyl triethoxysilane, gamma-(meth)acryloxypropyltrimethoxysilane, gamma(meth)acryloxypropyltriethoxysilane, and vinyl triacetoxysilane.

The amount of the compound to be grafted, e.g. of said silane compound is not critical and depends on the desired grafting degree. As an example, a silane compound may be grafted in an amount of 0.001 to 15 wt%, preferably 0.01 to 10 wt%, such as 0.01 to 5 wt% based on the weight of the grafted polymer composition to be grafted.

The amount of Compound of formula (I) as a free radical generating agent is not critical and depends on the desired grafting degree. As an example only, the amounts exemplified above under "1.Crosslinking of polymers" can be used.

The invention further provides a grafted polymer composition obtainable by a grafting process of the invention as defined above. Said grafted polymer composition may be further treated. Silane-grafted polymers may e.g. be further crosslinked, in a well known manner by hydrolysing said silane groups and subsequently condensing using a silanol condensation catalyst. As such catalysts e.g. known tin based catalyst and aromatic organic sulphonic acids can be used. Also the silane-grafted and crosslinked products are within the scope of the invention.

### 3. Visbreaking of polymers

A further embodiment of the modification method of the invention is a visbreaking of a polymer by radical reaction using a free radical generating agent, wherein said free radical generating agent is Compound of formula (I).

The "visbreaking of a polymer" is a well known modification method for modifying the melt flow rate (MFR) of a polymer.

One preferable polymer for visbreaking are homopolymers of propylene, random copolymers of propylene or heterophasic copolymers of propylene. Suitable homo, random and heterophasic polypropylenes (PP) may be produced using common polymerisation processes known in the art. Also the polypropylene polymer may be produced in a single- or multistage process of propylene or propylene and one or more comonomers, such as ethylene or higher alpha olefin. The polymerisation can be effected as bulk polymerisation, gas phase polymerisation, slurry polymerisation, solution polymerisation or any combinations thereof, in any order, using conventional catalysts including Ziegler-Natta, single site, such as metallocene or non-metallocene, catalysts. In case of heterophasic copolymer of polypropylene the matrix of homo or random copolymer can be produced e.g. in a single stage or as a multistage process described above and the elastomeric (rubber) part of the propylene copolymer can be produced as a in-situ polymerisation e.g. in a separate reactor, e.g. gas phase reactor in the presence of the matrix polymer produced in the previous stage(s). Alternatively the elastomeric part of propylene can be incorporated to the matrix phase material by compounding. The heterophasic copolymer of polypropylene thus comprises a matrix phase of homo- or copolymer of polypropylene and an elastomeric propylene copolymer dispersed in the matrix.

The amount of free radical generating agent used for visbreaking is not critical and depends on the level of desired MFR modification. Visbreaking can be carried out in known manner using very well known process and conditions documented in the literature.

### II. Polymer composition

The invention thus provides also a polymer composition comprising a free radical generating agent which is Compound of formula (I) as defined above. The polymer composition comprises at least one polymer component and at least one free radical generating agent, which is a compound of formula (I) including the preferred embodiments and subgroups thereof, as defined above or in claim 1 or dependent claims thereof.

Also a process for producing a polymer composition is provided, wherein the Compound of formula (I) is added to a polymer composition.

The amount of the Compound of formula (I) can naturally vary depending on the desired modification method. Examples of the amounts are given e.g. above under "1. Crosslinking of polymers". Moreover, the polymer composition of the invention may additionally, comprise further free radical generating agent(s), such as another Compound of formula (I).

Furthermore, polymers suitable for the polymer composition of the invention are not limited and include e.g. polymers 1) to 4), preferably 1) LDPE homo and copolymers, as described above under "1.Crosslinking of polymers".

The polymer composition of the invention can be in a well known powder or pellet form, or in a form of polymer melt. Polymer powder of the invention may i.a. be obtained directly from the polymerization process, optionally further processed, e.g. sieved, and/or optionally treated or mixed with further components.

The polymer pellets of the invention can be produced in a well known manner. In one process for forming the pellets of the invention the polymer powder or melt obtained from a polymerization process, or alternatively polymer pellets, may optionally be mixed with other components and pelletised e.g. by extrusion in a known pelletising equipment. The Compound of formula (I) may be added 1) to a mixture of a polymer composition prior to the pelletising step or 2) after the pelletising step by adding the Compound of formula (I) to the preformed pellets by mixing and/or impregnating, optionally in a carrier medium, to obtain the polymer pellets of the invention.

Alternatively, Compound of formula (I) can added to the polymer powder or polymer pellets directly in a production line of an end product, such as cable production line. Addition can be effected in a mixing step preceding the end product formation step or during the end product formation step, e.g. cable extrusion step. Such addition can be effect to the polymer composition in powder or pellet form or to a melt of said powder or pellets which melt may optionally contain further components.

Accordingly the invention provides a polymer powder, polymer pellets or a polymer melt comprising a polymer composition and a free radical forming agent, wherein said free radical forming agent is Compound of formula (I).

In one preferable embodiment of the pellets or powder of the invention, said pellets or powder are in a package, such as a container including boxes and bags. Such containers can be supplied for further use. E.g. an end producer, e.g. a cable producer, can then use the pellets of the invention as such for polymer modification step without need to add any free radical generating agent.

Moreover, the polymer composition of the invention may further contain other components, such as other polymers as mentioned above, or additives, such as stabilizers.

In one preferable embodiment of the polymer composition of the invention said polymer composition further comprises additives, such as one or more of antioxidants, stabilisers, processing aids, scorch retardants, crosslinking boosters or water tree retardants, or any mixtures thereof. As antioxidant, sterically hindered or semi-hindered phenols, optionally substituted with functional group(s), aromatic amines, aliphatic sterically hindered amines, organic phosphates, thio compounds, and mixtures thereof, can be mentioned. Typical cross-linking boosters may include compounds having a vinyl or an allyl group, e.g. triallylcyanurate, triallylisocyanurate, and di-, tri- or tetra-acrylates. As further additives, flame retardant additives, acid scavengers, fillers, such as carbon black, and voltage stabilizers can be mentioned. All the above mentioned additives are well known in polymer field. Such compositions are very useful for wire and cable applications, such as for cables of the invention discussed below.

The invention further provides (a) a process for crosslinking a polymer composition as herein described via free radical formation wherein the crosslinking is effected by producing methane as a decomposition product of said crosslinking step in an amount of less than 300 ppm (weight), when determined according to a method as described below under "GC-analysis protocol". Preferably said crosslinking step is carried out without producing methane as a decomposition product of said crosslinking step. A preferable embodiment of said process for crosslinking a polymer composition by radical reaction using a free radical generating agent results in a crosslinked polymer composition and produces a CH₄ content of less than 300 ppm (weight), preferably of less than 200 ppm (weight), preferably of less than 100 ppm (weight), more preferably of from 0 to 50 ppm (weight) when measured as defined below under "GC-analysis protocol".

The invention provides also independently (b) a process for crosslinking a polymer composition via free radical formation using one or more free radical generating agents, wherein the crosslinking is carried out using a Compound, of formula (I). A preferred crosslinking process is the above process (b). In demanding end applications it is preferred that said crosslinking according to process (b) is carried out using a free radical generating agent which does not produce methane (CH₄) during crosslinking step.

Preferably the crosslinking process is carried out under "crosslinking conditions" which means herein under free radical agent decomposing conditions. E.g. an elevated temperature is typically used for accelerating the decomposition of the radical and thus the crosslinking step. Moreover, "in the absence of CH₄ originating from said free radical generating agent" means that the free radical generating agent does not result in CH₄ as a decomposition product thereof during the crosslinking step.

Said crosslinking process is further defined above under "1.Crosslinking of polymers".

### III. End Applications

### 1. Article

The new principle of the invention is highly feasible in wide variety of end applications of polymers.

Accordingly, the invention further provides an article comprising the polymer composition of the invention as defined above and below, preferably under above "II. Polymer composition" or in claims, which is referred herein below as "polymer composition of the invention".

### 1.1 Cable

In one preferable embodiment said article of the invention is a cable comprising a conductor surrounded with one or more layers, wherein at least one layer comprises said polymer composition of the invention.

The term "conductor" means herein above and below that the conductor comprises one or more wires. Moreover, the cable may comprise one or more such conductors. Preferably the conductor is an electrical conductor.

In one embodiment of the cable of the invention at least one layer is an insulation layer which comprises said polymer composition of the invention.

In another embodiment of the cable of the invention at least one layer is a semiconductive layer comprising said polymer composition of the invention. "Semiconductive layer" means herein that said layer comprises carbon black and has a volume resistivity of 100 000 Ω-cm or below when measured at 23°C or 90°C, or, when measured according to ISO 3915 using a plaque, has a volume resistivity of 100 Ω-cm or below at 23°C, or of 1000 Ω-cm or below at 90°C.

In further embodiment, the cable of the invention comprises a jacketing layer and optionally one or more layers selected from an insulation layer and semiconductive layer surrounded by said jacketing layer, wherein said jacketing layer comprises said polymer composition of the invention.

As one further embodiment of the cable of the invention, a low voltage cable is provided which comprises an insulation layer and optionally a jacketing layer, wherein said insulation layer comprises said polymer composition of the invention.

As a further embodiment of the cable of the invention, a power cable is provided which comprises at least an inner semiconductive layer, insulation layer and an outer semiconductive layer, in that order, optionally surrounded by a jacketing layer, wherein at least one of said layers, preferably at least the inner semiconductive layer and insulation layer, comprises said polymer composition of the invention.

In the context of the present invention, a low voltage cable is a cable operating in voltages 1 kV or below. A power cable is defined to be a cable transferring energy operating at any voltage, typically operating at voltages higher than 1 kV. The voltage applied to the power cable can be alternating (AC), direct (DC), or transient (impulse). In a preferred embodiment, the power cable prepared according to the present invention is operating at voltages higher than 6 kV and are known i.a. as medium voltage (MV), high voltage (HV) and extra high voltage (EHV) power cables, which terms have well known meaning and indicate the operating level of such cable.

Said outer semiconductive layer of said power cable of the invention can be non-strippable, i.e. bonded and non-peelable, or strippable, i.e. non-bonded and peelable. Said terms have well known meanings in the wire and cable field.

### 2 Preparation process of an article

The present invention further provides a process for producing an article by using said polymer composition of the invention.

### 2.2. Preparation process of a cable

A preferable embodiment of the process for preparing an article of the invention is a process for producing a cable comprising steps of applying, preferably by (co)extrusion, one or more layers on a conductor, which layers comprise a polymer composition, wherein at least one layer comprises said polymer composition of the invention.

The term "(co)extrusion" means herein that in case of two or more layers, said layers can be extruded in separate steps, or at least two or all of said layers can be coextruded in a same extrusion step, as well known in the art.

In said process of the invention the components of a layer material are mixed in a separate mixer before being introduced to the extruder for producing said layers or are added directly to an extruder and mixed therein before forming a layer. Additives and further components can be added during the mixing step. The mixture in extruder is subjected to an elevated temperature, typically above the melting point of the polymer components and then (co)extruded on a conductor in a manner very well known in the field. E.g. conventional extruders and mixers may be used in the process of the invention.

The above described polymer powder, polymer pellets or melt of the invention, which comprise said polymer composition of the invention, can each equally be used in said process for preparing cables and they can be prepared prior their use in the cable preparation step or they can be prepared directly in a cable production line during a cable manufacturing process, as described above e.g. under "II. Polymer composition". Accordingly, 1) preformed powder or pellets of a polymer composition of the invention comprising Compound of the invention, may be subjected to the cable production line; or 2) said Compound of the invention may be added together with pellets or powder to a mixing step before forming the cable layer(s). Such mixing step can be a separate step in a separate mixing device arranged in the cable production line to precede the cable layer formation step, e.g. an extrusion step. Alternatively, Compound of formula (I) can be added during the layer formation step e.g. in an extruder, whereby it can be introduced to the extruder together with or after the addition of polymer powder or polymer pellets. The addition point in an extruder is not limited, whereby the Compound of formula (I) can be added at the inlet of the extruder or at a later feed point arranged along the extruder. Accordingly the addition of Compound of formula (I) may take place at the time the polymer material is in solid non-molten, partly molten or molten state, i.e. a melt mixture. The obtained molten mixture of a layer material is then (co)extruded on to a conductor to form a cable layer. In a preferred cable preparation process of the invention a low voltage cable or, more preferably, a power cable of the invention as defined above under 1.1. Cable is produced. The obtained cable can be further processed for the end use application.

A crosslinking process (C2) for crosslinking a cable by radical reaction using one or more free radical generating agents, comprises the step of: applying one or more layers comprising a polymer composition on a conductor, wherein at least one layer comprises one or more free radical generating agents, crosslinking by radical reaction said at least one layer comprising said free radical generating agent(s), and recovering the crosslinked cable in a conventional manner for further use; wherein said crosslinking is carried out in the presence of Compound of formula (I) as a free radical generating agent.

In above crosslinking process of the invention crosslinking conditions can vary depending i.a. on the used materials and cable size. The crosslinking of the invention is effected e.g. in a known manner preferably in an elevated temperature. Preferably the lowest temperature in a cable layer during the crosslinking step is above 140°C, more preferably above 150°C, such as 160-210°C. The crosslinking may be carried out in a liquid or gas medium, such as in an inert gas, such as N₂, atmosphere. The pressure during the crosslinking step of the invention is typically up to 20 bar, preferably up to 13 bar, such as 10-15 bar, in inert atmosphere. Said crosslinking step of the invention is also described above under "1. Crosslinking of polymers" and above under "II. Polymer composition".

A further preferable embodiment of the crosslinking process of the invention comprises a further step of cooling the crosslinked cable preferably under pressurized conditions in a cooling medium e.g. in gas or liquid, such as N₂, oil or water. The cooling is effected in a cooling zone, which may be optionally integrated with the preceding crosslinking zone, e.g. in a known vulcanization tube. As an example only, continuous catenary vulcanization (CCV) tube can be mentioned. The temperature at the layer closest to conductor is typically below 200°C, e.g. 160-190°C, at the beginning of the cooling zone/step. The pressure during the cooling step of the invention is typically kept above atmospheric pressure, e.g. up to 20 bar, preferably up to 13 bar, such as 10-12 bar. The cable is removed from the pressurized cooling step, when the temperature of the cable layers is clearly below the melting point of the polymer layer material thereof. Accordingly, the crosslinked cable of the invention may leave the pressurized cooling step of the invention e.g. when the temperature of the conductor of said cable is below 110°C depending on the layer polymer material, preferably between 70-90°C, at the exit of the pressurized cooling zone.

The crosslinking and cooling step is normally carried out under pressurized conditions to prevent the formation of voids due to volatile decomposition products of e.g. peroxides. In a preferable embodiment of the crosslinking process of the invention thus enables to remove the crosslinked and cooled cable from the pressurized cooling zone in a temperature higher than in the prior art, when measured from the conductor. Also preferably, the cooling may be effect at lower pressures compared to prior art.

Optionally, if desired, the crosslinked cable of the invention may be subjected to an additional non-pressurised cooling step after said pressurized cooling step, for further cooling of the cable.

The cable preparation process of the invention optionally comprises a further recovering step of the cable coming from the cooling step. Recovering may be effected by winding the cable on a cable drum in a known manner.

In a further embodiment of the process of the invention the cable obtained from the cooling step and optionally recovered, e.g. wound to a cable drum, may optionally be subjected, if needed in some applications, to a subsequent degassing step i.a. for removing or reducing any volatile decomposition products possibly resulting from said crosslinking step of the invention. In said degassing step the cable of the invention is preferably exposed either in ambient or elevated temperature for a period of time. As an example only, said degassing temperature may be e.g. 50-80°C, for a time period of one to four weeks. In one embodiment of the crosslinking process said degassing step may be shortened considerably or even avoided due to decreased level of said volatile by-products.

The cable of the invention produced by the above process of the invention may finally be further processed, e.g. protected with a protective layer, and/or optionally covered by a jacketing layer in a subsequent finishing step in a known manner and recovered for the end use thereof.

In one embodiment of a crosslinking process of the invention a crosslinked power cable is produced which is selected from a crosslinked MV cable, wherein the lowest degree of crosslinking in a cable layer(s) meets the requirements as specified in IEC 60502, or a crosslinked HV cable, wherein the lowest degree of crosslinking in a cable layer(s) meets the requirements as specified in IEC 60840, which specifications are well known in the W&C field.

The advantageous Compounds of the invention are preferable free radical generating agents which can be used for improving the quality of the products, e.g. in cable production processes. Due to the present invention the amount of voids in polymer products, such as cable layers can be reduced or even avoided, since less or no volatile decomposition products are formed from e.g. when Compound of the invention is used for modifying the polymer. Moreover, the invention also enables to improve the processability of a cable, i.a. in terms of safer and faster processing. E.g. the crosslinking process of the invention can be faster and/or more economical, since both cooling and/or degassing steps may be carried out in a reduced time and/or in a less energy consuming manner, if desired.

### Determination methods

Unless otherwise stated the below determination methods were used to determine the properties defined generally in the description part and claims and in the experimental part.

### Melt Flow Rate

The melt flow rate (MFR) is determined according to ISO 1133 and is indicated in g/10 min. The MFR is an indication of the flowability, and hence the processability, of the polymer. The higher the melt flow rate, the lower the viscosity of the polymer. The MFR is determined at 190 °C for polyethylenes and may be determined at different loadings such as 2.16 kg (MFR₂) or 21.6 kg (MFR₂₁). The MFR is determined at 230 °C for polypropylenes.

### Density

The density was measured according to ISO 1183D. The sample preparation was executed according to ISO 1872-2.

### Gel Content

The gel content was determined according to ASTM D 2765-01, method A using a crosslinked sample which consists of the polymer composition under test and prepared according to "**Preparation of samples, crosslinking**" **below**.

Gel content on a cable is carried out using ASTM D 2765-01 method B.

### Methods A and B give comparable results.

### GC-Analysis protocol

In definitions of the Compounds, Polymer compositions, cables and preparation process and modification methods thereof as defined above and in claims below, the volatile, e.g. CH₄, content given in ppm (weight) or as "absent" is determined by gas chromatography (GC) from a sample which is modified, e.g. crosslinked.

The test is used to determine the produced volatiles, e.g. methane, content of a free radical generating agent. The test free radical generating agent is used in such an amount with which a crosslinking degree expressed as gel content of 50 % was achieved, preferably gel content of at least 50%. Crosslinking conditions of the sample are not critical and may be effected e.g. as described below under

### "Preparation of samples, crosslinking"

Volatiles are measured by taking a sample specimen of 1 g with a thickness of 1.5 mm from a modified, e.g. crosslinked composition, e.g. a plaque or cable. In the case of a cable comprising a crosslinked layer(s), the sample specimen is taken from a layer material of a crosslinked and cooled cable sample that is taken at the exit of a crosslinking/cooling zone, such as at the exit of a vulcanisation tube, after pressurised cooling step in a manner known for a skilled person.

The collection of volatiles from said sample specimen (to a head space bottle, see below) is started within one hour after the modification step is stopped, or in case of a crosslinked and cooled cable, within one hour after the cable sample is taken at the exit of a crosslinking/cooling zone.

A sample specimen of a thickness of 1.5 mm and of a weight of 1 g is cut from the middle of a plaque which is 100 mm wide and 100 mm long. The obtained sample (specimen) is placed in a 120 ml head space bottle with an aluminium crimp cup with seal and heat treated at 60 ° C for 1.5 h for collecting any gaseous volatiles present in said sample. Then 0.3-0.5 ml of the gas captured in the sample bottle is injected into a gas chromatograph, wherein the presence and content of the volatiles, e.g. methane, which are desired to be measured in a known manner. Double samples are analysed and a "zero-sample" without free radical generating agent/modification is used as a reference. The instrument used herein was a Varian 3400 with a Al₂O₃/Na₂SO₄ -column of 0,53mm x 50m, supplied by Chrompack.

### Specimen from a cable

A sample specimen of a thickness of 1.5 mm and of a weight of 1 g is cut in an axial direction from said cable sample from the middle distance (in radial direction) of the polymer layer(s) ring surrounding the conductor of said cable sample (i.e. at the distance of ½ radius of said cable layer ring). The collection and determination of volatiles was carried out as described above.

### Specimen from a plaque

The volatile, e.g. CH₄, content given in ppm (weight) or as "absent" is determined by gas chromatography (GC) from a sample plaque which is modified, e.g. crosslinked according to the protocol described in the section entitled "Preparation of samples, crosslinking" above. The test composition contains 2 parts test peroxide and 100 parts test polymer (i.e: sufficient to cause a degree of cross-linking of 50% or more).

A sample specimen of a thickness of 1.5 mm and of a weight of 1 g is cut from the middle of a plaque which is 100 mm wide and 100 mm long. The collection and determination of volatiles was carried out as described above.

### Materials

In each test for references and for examples of this application the test arrangement for the reference polymer, i.e. the polymer without any added additive such as organic peroxide, and for the tested compositions, i.e. the reference polymer containing the organic peroxide, was the same.

### The unsaturated polymer: The polymer is a poly (ethylene -co-1,7-octadiene)

### Poly (ethylene -co-1,7-octadiene) manufacture

Ethylene was compressed in a 5-stage precompressor and a 2-stage hyper compressor with intermediate cooling to reach an initial reaction pressure of ca. 2950 bar. The total compressor throughput was ca. 30 tons/hour. In the compressor area approximately 120 kg propylene / hour was added as chain transfer agent to maintain an MFR of 3.2g / 10 min. Here also 1,7-octadiene was added to the reactor in amount of ca. 50 kg/h. The compressed mixture was heated to approximately 165 °C in a preheating section of a front feed three-zone tubular reactor with an inner diameter of ca. 40 mm and a total length of ca. 1200 meters. A mixture of commercially available peroxide radical initiators dissolved in isododecane was injected just after the preheater in an amount sufficient for the exothermal polymerization reaction to reach peak temperature of ca. 280 °C after which it was cooled to approx 250 °C. The subsequent 2nd and 3rd peak reaction temperatures were ca. 280 °C and ca. 270 °C, respectively, with a cooling in between down to approximately 250 °C. The reaction mixture was depressurized by a kick valve, cooled and polymer was separated from unreacted gas.

The obtained polymer had a total number of C-C carbon double bonds of 1.286/1000 C and the number of vinyl groups was 0.994 vinyl groups/1000 C. The density of the material was 920 kg/m³ and MFR(2.16 kg) = 3.2 g/10 min.

The above unsaturated polymer was used in testing the examples of the invention containing compounds (I) of the invention as the crosslinking agent, comparative examples with dicumulperoxide as the crosslinking agent and the reference example containing no crosslinking agent.

The commercial reference organic peroxide, dicumyl peroxide, was supplied by AkzoNobel.

### Preparation of samples, impregnation

The test polyethylene pellets were ground to a fine powder in a Retsch grinder with a 1.5 mm sieve. The powder obtained was impregnated with the test peroxide dissolved in a pentane solution until the pentane had evaporated to give a dry powder of the test peroxide and the test polymer. The content of the test composition was 3 parts test peroxide and 100 parts test polymer when the gel content of the crosslinked test composition was tested as described below. The content of the test composition was 2 parts test peroxide and 100 parts test polymer when the volatiles content was determined as described in the GC-analysis protocol.

### Preparation of samples, crosslinking

The test plaques had the following dimensions and crosslinking cycle. The plaques were 100 mm long, 100 mm wide, and 0.1 mm thick when used for determination of the gel content as described below, and 100 mm long, 100 mm wide, and 1.5 mm thick when the volatiles content was determined as described in the GC-analysis protocol below. The crosslinking was conducted in a Specac press, where the composition was kept at 120 °C for 1 min at 5 bar, then the temperature was increased with 60 °C/min for 1 min to reach 180 °C at 5 bar, and kept at 180 °C at 5 bar for 12 min, followed by cooling to ambient temperature over 30 min at 5 bar.

### EXAMPLES

### Example 1

### Preparation of Di-(1-methyl-1-phenylundecyl) peroxide (R¹, R¹, phenyl; R², R²' = methyl; R³, R³' = decyl)

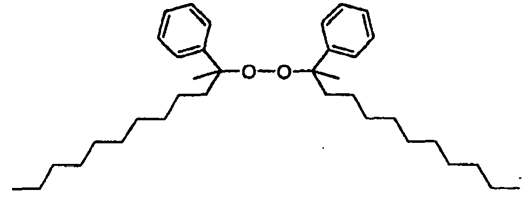

(Ib)

### A. 1-methyl-1-phenylundecyl alcohol

To a suspension of 2.43 g (0.1 mol) magnesium turnings in 10 ml of diethyl ether was added 0.1 ml of 1,2-dibromoethane and the mixture was stirred. 22.17 g (0.1 mol) of 1-bromodecane in 20 ml diethyl ether was added dropwise and the mixture was refluxed for 15 minutes, then cooled. 9.61 g (0.08 mol) of acetophenone in 20 ml diethyl ether was added while cooling on ice bath. The ice bath was removed and the reaction mixture stirred at room temperature for 30 minutes. The mixture was then poured into a slurry of 30 g ammonium chloride in 150 ml water and 100 g ice while stirring vigorously. The mixture was filtered, the ether layer separated and the aqueous layer extracted twice with 50 ml of ether. The organic layers were combined, washed with water, 10% NaHSO₃, brine, dried and evaporated to give 22.48 g of clear oil. The oil was purified with dry column chromatography using pentane. The eluant was evaporated giving 17.22 g (82%) of 1-methyl-1-phenylundecyl alcohol as a viscous colorless oil.

### B. 1-methyl-1-phenylundecyl hydroperoxide

10.50 g (0.04 mol) of 1-methyl-1-phenylundecyl alcohol was dissolved in 50 ml of dichloromethane, cooled in ice bath, 10.6 ml (12.29 g, 0.08 mol) of trimethylsilyl bromide was added and the mixture stirred for 1 h under protection from moisture. The solution was diluted with 100 ml ether and washed four times with 50 ml water, brine, dried and evaporated to give crude 2-phenyl-2-bromododecane. 35 ml of 2.3 M hydrogen peroxide in THF (0.08 mol) was added to the 2-phenyl-2-dodecyl bromide and the mixture was cooled on ice bath. 8.84 g (0.04 mol) of silver trifluoroacetate was added. 70 ml of conc. NaHCO₃ was added and the mixture filtered. The reaction flask and the filter cake was rinsed with diethyl ether. The aqueous phase was separated and the organic phase washed with conc. NaHCO₃, 50 ml water, brine, dried and evaporated to give an oil. The oil was purified by flash chromatography using 2:8 ether:pentane as eluent. The yield of 1-methyl-1-phenylundecyl hydroperoxide was 30%.

### C. Di-(1-methyl-1-phenylundecyl) peroxide

0.942 g (3.6 mmol) of 1-methyl-1-phenylundecyl alcohol was dissolved in 5 ml of dichloromethane, 1 ml of trimethylsilyl bromide (7.2 mmol) was added and the mixture stirred for 1 h under protection from moisture. The solution was diluted with 15 ml of diethyl ether, washed with water (3x10 ml), 15 ml brine, dried and evaporated to give 1.18 of crude 2-phenyl-2-bromododecane. 795 mg of silver trifluoroacetate (3.6 mmol) was dissolved in 5 ml THF. To the crude bromide was added 500 mg of 1-methyl-1-phenylundecyl hydroperoxide (1.8 mmol) dissolved in 10 ml THF. This mixture was cooled in ice-salt bath to -15°C and the silver trifluoroacetate solution added with a pipette. 2 ml of brine was then added, followed by 10 ml conc. NaHCO₃. The reaction mixture was stirred and filtered. The reaction flask and the filter cake were rinsed with 15 ml diethyl ether. The aqueous phase was separated and the organic phase washed with conc. NaHCO₃, 15 ml water, 15 ml brine, dried and evaporated to give 1.40 g of a yellowish oil. Purification was done using a 1:9 ether:pentane mixture as eluent. The yield was 409 mg (43%). ¹³C-NMR (CDCl₃) δ 14.33, 22.91, 23.96, 24.06, 24.19, 29.55, 29.72, 29.82, 30.24, 32.13, 40.68, 40.97, 84.18, 126.16, 126.69, 127.86, 145.59, 145.71

### Example 2

### Preparation of Di(1-methyl-cyclohexyl) peroxide

### (R¹ = methyl; R² + R³ form together with C¹ a cyclohexyl ring and R^{1'} = methyl; R^{2'} + R^{3'} form together with C^{1'} a cyclohexyl ring)

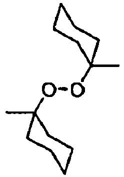

### A. Di(1-methyl-cyclohexyl)peroxide

1-methylcyclohexanol (30 g, 0.26 mol) was placed in a 100mL three necked round bottomed flask and was stirred. The flask was cooled in a brine/ice bath, dropping funnel fitted and fitted with a static N₂ supply. The dropping funnel was charged with 98% sulfuric acid (16.14 ml) and water (6.45 ml) giving a 70% sulfuric acid solution. This was added dropwise to the 1-methylcyclohexanol and stirring continued to give a viscous brown mixture. The bath was recharged with ice/brine, dropping funnel rinsed with water and recharged with 35% hydrogen peroxide (6.98mL, 0.125ml) and added dropwise. The solution separated into two phases. Cyclohexanol (150mL) was added and the mixture was transferred to a separating funnel. The aqueous fraction was extracted with another portion of cyclohexane (150ml) and the combined organic fractions washed with 1M NaOH (2x100mL), water (2x150mL), dried and evaporated to give a viscous colourless oil. (12.98g). The oil was sorbed onto silica gel then placed on a silica gel column and eluted with cyclohexane. After evaporation at reduced pressure 0.7 g colourless oil of di(1-methyl-cyclohexyl) peroxide was obtained. ¹³C-NMR (CDCl₃) δ 22.45, 25.01, 25.95, 35.39, 78.58

### Example 3

### Preparation of Di(1-methyl-cyclopentyl) peroxide

(R¹ = methyl; R² + R³ form together with C¹ a cyclopentyl ring and R^{1'} = methyl; R^{2'} + R^{3'} form together with C^{1'} a cyclopentyl ring)

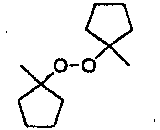

### A. Di(1-methyl-cyclopentyl) peroxide

A 250 ml tri-neck round bottom flask was equipped and a 50 ml addition funnel and the flask was cooled <0C. 30g of 1-methylcyclopentanol (0.3 M, 1 EQ) was added to the flask. 70% H₂SO₄ solution was prepared and cooled in an ice bath. The H₂SO₄ (12.71 ml, 0.91 M, 3 EQ) was added drop wise over 15 minutes and the reaction mixture was stirred for ~2.5 hours in order to allow all the 1-methylcyclopentanol to dissolve. With the reaction stirring, 8.11ml of H₂O₂ 35% (wt) (0.24 M, 0.8 EQ) was added drop wise over 15 minutes. The reaction was left stirring overnight. The reaction mixture was transferred to a separation funnel and extracted three times with 50 ml of pentane each time. Organic layers were collected and the aqueous was set aside. The organic layers were extracted 3 times with 50 ml of 1N NaOH each time to remove excess acid. The organic layer was collected, dried and concentrated. The residue was purified by chromatography on a silica column using pentane as the mobile phase. The product fractions were concentrated to yield 973 mg of di(1-methyl-cyclopentyl) peroxide as a colorless oil. ¹³C-NMR (CDCl₃) δ 24.43, 24.75, 37.13, 89.23

### Gel content

The gel content of the LDPE copolymer prepared as described above was determined according to the method above and the results are shown below (Table 1.)

**Table 1. Gel content**

| Example | Gel content (%) |
|---|---|
| Reference polymer without peroxide | 0 |
| Ib | 51 |
| Ia | 62 |
| Ia' | 82 |

### GC-Analysis

GC-analysis was performed to evaluate the amount of formed CH₄. The example is compared to a sample using dicumyl peroxide, which represent the conventional solution used today. The results are presented below (Table 2).

**Table 2. GC-analysis of the CH₄ content.**

| Example | CH₄ content (ppm) |
|---|---|
| Dicumyl peroxide | 719 (gel content 93%) |
| Ia' | <5* (gel content 84%)* |
| * at values less than 5 ppm the amount of methane is so small that noise masks an accurate reading. Value less than 5ppm are considered to represent non methane formed therefore. | |

### Preparation of a crosslinked cable of the invention:

A power cable comprising an inner semiconductive layer, an insulation layer and an outer semiconductive layer for experimental testing is prepared by coextruding on a conductor said layers in given order using a conventional extruder line and conventional extrusion conditions.

The layer materials are conventional polymer grades and each layer comprises a peroxide compound of the invention as a crosslinking agent.

The semiconductive material used in the cable, both as inner and outer semicon, is a poly(ethylene-co-butylacrylate) polymer (with a butylacrylate content of 17wt%) containing 40 wt% of a furnace black. The composition is stabilised with an antioxidant of the polyquinoline type and contains 1 wt% of the peroxide of the invention as a crosslinking agent.

The middle insulation layer is formed of low density polyethylene LDPE (MFR₂=2 g/10 min) containing 2 wt-% of the peroxide of the invention and 0.2 wt-% of 4,4'-thiobis(2-tert.-butyl-5-methylphenol).

The obtained cable is immediately after extrusion subjected to a conventional vulcanisation tube and crosslinked in a known manner using well known crosslinking conditions. After crosslinking the cable is then cooled in cooling zone of said vulcanisation tube at a desired pressure and temperature. The cooling step is stopped when the desired temperature measured from the conductor is achieved. Typically the cooling step can be effected in a lower pressure and/or the cooling step can be stopped in a higher temperature at conductor compared to corresponding cable crosslinked to a same gel content, but using dicumylperoxide as the crosslinking agent. The crosslinked and cooled layer is wound to a cable drum and transferred to a degassing step to remove the volatile(s) content, if any. This step can typically be done in a lower temperature and/or a shorter period compared to corresponding cable crosslinked to a same gel content, but using dicumylperoxide as the crosslinking agent.

## Claims

1. A polymer composition comprising
- at least one polymer component, e.g. in a form of (1) polymer powder, (2) polymer pellets or (3) a polymer melt. whereby said (1) polymer powder or, preferably, (2) polymer pellets are optionally contained in a container; and
- at least one free radical generating agent
wherein the at least one free radical generating agent is a compound which is an organic peroxide of formula (I) 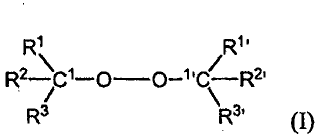
wherein R¹ and R^{1,} are the same and each represents methyl; and
R² and R³ together with C¹ atom to which they are attached form a saturated or partially unsaturated mono- or bicyclic (C4-C14)carbocyclic ring,
and R^{2,} and R^{3,} together with the carbon atom (C¹') to which they are attached form a saturated or partially unsaturated mono- or bicyclic (C4-C14)carbocyclic ring whereby the ring system formed by R² and R³ together with C¹ is preferably identical to a ring system formed by R^{2,} and R^{3,} together with C¹'; or
R¹ and R^{1,} are the same and represent an unsubstituted, monocyclic (C5-C7)aryl;
R² and R^{2,} are the same and are methyl; and
R³ and R^{3,} are the same and are straight chain (C6-C30)alkyl.

2. A composition as defined in any of the preceding claims, wherein R² and R³ together with carbon atom (C¹) to which they are attached and/or wherein R^{2,} and R^{3,} together with carbon atom (C¹') to which they are attached form an unsubstituted, saturated monocyclic (C5-C8)carbocyclic ring.

3. A composition as defined in any of the preceding claims, wherein the ring system formed by R^{2,} and R^{3,} together with the carbon atom (C¹') to which they are attached is same as the ring system formed by R² and R³ together with the carbon atom (C¹) to which they are attached.

4. A composition as defined in claim 1, wherein the free radical generating agent is a compound selected from any of
- Di-(1-methyl-1-phenylundecyl)peroxide
- Di-(1-methyl-1-phenylheptyl)peroxide
- Di(1-methyl-cyclohexyl)peroxide or
- Di(1-methyl-cyclopentyl)peroxide.

5. A process for crosslinking a polymer composition via radical reaction using a free radical generating agent, **characterized in that** a radical reaction is initiated, e.g. by heating, in a polymer composition comprising a free radical generating agent which is a compound as defined in claims 1 to 4 to thereby effect crosslinking.

6. A crosslinkable cable which comprises a conductor which is surrounded by one or more layers comprising a polymer composition, **characterized in that** at least one layer comprises a polymer composition as defined in any of claims 1 to 4.

7. A crosslinkable cable as defined in claim 6 which comprises at least one of an insulation layer, semiconductive layer or jacketing layer which comprises a polymer composition as defined in any of claims 1 to 4; or
- a low voltage cable comprising a conductor surrounded by an insulation layer and optionally a jacketing layer, wherein said insulation layer comprises a polymer composition as defined in any of claims 1 to 4; or
- a power cable comprising an electrical conductor surrounded by one or more layers comprising at least an inner semiconductive layer, insulation layer and an outer semiconductive layer, in that order, and optionally surrounded by a jacketing layer, wherein at least one of said layers comprises, preferably at least inner semiconductive layer and insulation layer, a polymer composition as defined in any of claims 1 to 4.

8. A process for crosslinking a cable by radical reaction, comprising:
- applying one or more layers comprising a polymer composition as claimed in claim 1 to 4 on a conductor, and
- crosslinking by radical reaction said at least one layer comprising said free radical generating agent(s),
- subjecting the cable thus obtained to a further cooling step, wherein said crosslinked cable is cooled under pressurized conditions,
and, optionally, after said cooling step the crosslinked and cooled cable is subjected to one or more additional steps selected from:
- a non-pressurized cooling step, wherein the crosslinked and cooled cable is further cooled in a cooling medium,
- a recovering step, wherein the crosslinked cable is collected after the cooling step, preferably wound to a cable drum,
- a degassing step, wherein the content of volatile decomposition products(s) is reduced or removed, optionally at ambient or in elevated temperature, from said crosslinked cable obtained from said cooling and optional recovery step, and/or
- a finishing step, wherein the obtained crosslinked cable is finished in a conventional manner for further use;
**characterized in that** said crosslinking is carried out using a process as defined in claim 5.

9. A compound which is an organic peroxide of formula (III) 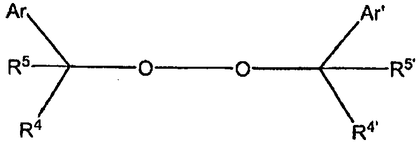 wherein Ar and Ar' independently represent a phenyl, benzyl or naphthyl group,
R⁴ and R^{4,} each are methyl; and
R⁵ and R^{5,} each independently represent a straight chain alkyl group having C6-30 carbon atoms, preferably 6 to 20, more preferably 6 to 12, carbon atoms;
or the compound di(1-methylcyclohexyl)peroxide.

10. A compound as defined in claim 9 which is selected from any of
- Di-(1-methyl-1-phenylundecyl)peroxide or
- Di-(1-methyl-1-phenylheptyl)peroxide.

## Patentansprüche

1. Polymerzusammensetzung, die folgendes aufweist:
- mindestens eine Polymerkomponente, z. B. in einer Form von (1) Polymerpulver, (2) Polymerpellets oder (3) einer Polymerschmelze, wobei das (1) Polymerpulver oder vorzugsweise die (2) Polymerpellets optional in einem Behälter enthalten sind; und
- mindestens ein freie Radikale erzeugendes Mittel,
wobei das mindestens eine freie Radikale erzeugende Mittel eine Verbindung ist, die ein organisches Peroxid mit der Formel (I) ist: 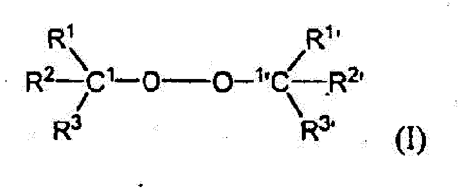
wobei R¹ und R^{1,} gleich sind und jeweils Methyl repräsentieren; und R² und R³ zusammen mit dem C¹-Atom, an das sie gebunden sind, einen gesättigten oder teilweise ungesättigten mono- oder bicyclischen (C4-C14)-carbocyclischen Ring bilden,
und R^{2,} und R^{3,} zusammen mit dem Kohlenstoffatom (C¹'), an das sie gebunden sind, einen gesättigten oder teilweise ungesättigten mono- oder bicyclischen (C4-C14)-carbocyclischen Ring bilden, wobei das Ringsystem, das durch R² und R³ zusammen mit C¹ gebildet wird vorzugsweise mit dem Ringsystem, das durch R^{2,} und R^{3,} zusammen mit C¹' gebildet wird, identisch ist; oder
wobei R¹ und R^{1,} gleich sind und ein unsubstituiertes, monocyclisches (C5-C7)-Aryl repräsentieren;
R² und R^{2,} gleich und Methyl sind; und
R³ und R^{3,} gleich und ein geradkettiges (C6-C30)-Alkyl sind.

2. Zusammensetzung nach einem der vorhergehenden Ansprüche, wobei R² und R³ zusammen mit dem Kohlenstoffatom (C¹), an das sie gebunden sind, und/oder wobei R^{2,} und R^{3,} zusammen mit dem Kohlenstoffatom (C¹'), an das sie gebunden sind, einen unsubstituierten, gesättigten monocyclischen (C5-C8)-carbocyclischen Ring bilden.

3. Zusammensetzung nach einem der vorhergehenden Ansprüche, wobei das Ringsystem, das durch R^{2,} und R^{3,} zusammen mit dem Kohlenstoffatom (C¹'), an das sie gebunden sind, gebildet wird, dasselbe Ringsystem ist, das durch R² und R³ zusammen mit dem Kohlenstoffatom (C¹), an das sie gebunden sind, gebildet wird.

4. Zusammensetzung nach Anspruch 1, wobei das freie Radikale erzeugende Mittel eine Verbindung ist, die irgendeinem aus dem Folgenden ausgewählt ist:
- Di-(1-methyl-1-phenylundecyl)peroxide
- Di-(1-methyl-1-phenylheptyl)peroxid
- Di-(1-methylcyclohexyl)peroxid oder
- Di-(1-methylcyclopentyl)peroxid

5. Verfahren zum Vernetzen von einer Polymerzusammensetzung mittels radikalischer Reaktion unter Verwendung von einem freien Radikale erzeugenden Mittel, **dadurch gekennzeichnet, dass** eine radikalische Reaktion, z. B. durch Erwärmen, in einer Polymerzusammensetzung, die ein freies Radikale erzeugendes Mittel umfasst, das eine Verbindung nach den Ansprüchen 1 bis 4 ist, initiiert wird, um dadurch eine Vernetzung zu bewirken.

6. Vernetzbares Kabel, das einen Leiter umfasst, der von ein oder mehreren Schichten umgeben ist, die eine Polymerzusammensetzung umfassen, **dadurch gekennzeichnet, dass** mindestens eine Schicht eine Polymerzusammensetzung nach einem der Ansprüche 1 bis 4 umfasst.

7. Vernetzbares Kabel nach Anspruch 6, das mindestens eine Isolierschicht, eine halbleitende Schicht oder Mantelschicht umfasst, die eine Polymerzusammensetzung nach einem der Ansprüche 14 umfasst; oder
- ein Niederspannungskabel, umfassend einen Leiter, der von einer Isolierschicht und optional einer Mantelschicht umgeben ist, wobei die Isolierschicht eine Polymerzusammensetzung nach einem der Ansprüche 1 bis 4 umfasst; oder
- ein Energiekabel, umfassend einen elektrischen Leiter, der von ein oder mehreren Schichten umgeben ist, umfassend mindestens eine innere halbleitende Schicht, eine Isolierschicht und eine äußere halbleitende Schicht, und zwar in dieser Reihenfolge, und der optional von einer Mantelschicht umgeben ist, wobei mindestens eine der Schichten, vorzugsweise mindestens die innere halbleitende Schicht und die Isolierschicht, eine Polymerzusammensetzung nach einem der Ansprüche 1 bis 4 umfassen.

8. Verfahren zum Vernetzen von einem Kabel durch radikalische Reaktion, umfassend:
- Aufbringen von ein oder mehreren Schichten, umfassend eine Polymerzusammensetzung nach einem der Ansprüche 1 bis 4, auf einen Leiter, und
- Vernetzen durch radikalische Reaktion von der mindestens einen Schicht, umfassend das (die) freie Radikale erzeugende(n) Mittel,
- Unterziehen von dem auf diese Weise erhalten Kabel einem weiteren Kühlschritt, wobei das vernetzte Kabel unter unter Druck gesetzten Bedingungen gekühlt wird,
und wobei optional nach dem Kühlschritt das vernetzte und gekühlte Kabel ein oder mehreren zusätzlichen Schritten unterzogen wird, ausgewählt aus:
- einem Kühlschritt unter nicht unter Druck gesetzten Bedingungen, wobei das vernetzte und gekühlte Kabel ferner in einem Kühlmedium gekühlt wird,
- einem Gewinnungsschritt, wobei das vernetzte Kabel nach dem Kühlschritt gesammelt wird, vorzugsweise auf eine Kabeltrommel gewickelt wird,
- einem Entgasungsschritt, wobei der Gehalt an flüchtigem(n) Zersetzungsprodukt(en), optional bei Raumtemperatur oder erhöhter Temperatur, aus dem vernetzten Kabel verringert oder entfernt wird, das aus dem Kühl- und dem optionalen Gewinnungsschritt erhalten wird, und/oder
- einem Endbearbeitungsschritt, wobei das erhaltene vernetzte Kabel auf eine herkömmliche Weise zur weiteren Verwendung endbearbeitet wird;
**dadurch gekennzeichnet, dass** das Vernetzen durchgeführt wird, wobei ein Verfahren nach Anspruch 5 verwendet wird.

9. Verbindung, die ein organisches Peroxid mit der Formel (III) ist: 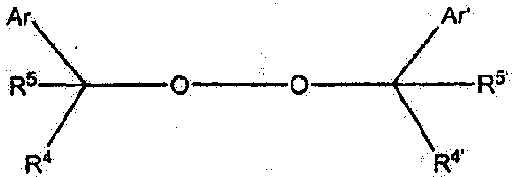
wobei Ar und Ar' unabhängig eine Phenyl-, Benzyl- oder Naphthylgruppe repräsentieren,
R⁴ und R^{4,} jeweils Methyl sind; und
R⁵ und R^{5,} jeweils unabhängig eine geradkettige Alkylgruppe mit C6-30 Kohlenstoffatomen, vorzugsweise 6 bis 20, besonders vorzugsweise 6 bis 12 Kohlenstoffatomen repräsentieren;
oder die Verbindung Di(1-methylcyclohexyl)peroxid ist.

10. Verbindung nach Anspruch 9, die aus dem Folgenden ausgewählt ist:
- Di(1-methyl-1-phenylundexyl)peroxid oder
- Di(1-methyl-1-phenylheptyl)peroxid.

## Revendications

1. Composition polymère comprenant :
- au moins un composant polymère, par exemple sous la forme (1) d'une poudre de polymère, (2) de pellets de polymère ou (3) d'une masse fondue de polymère, de sorte que ladite poudre de polymère (1) ou, de préférence, les pellets de polymère (2) soient éventuellement contenus dans un récipient ; et
- au moins un agent générateur de radicaux libres,
dans laquelle le au moins un agent générateur de radicaux libres est un composé qui est un peroxyde organique de formule (I) : 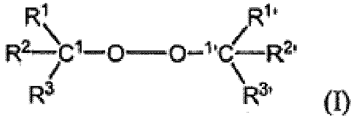
dans laquelle R¹ et R^{1,} sont identiques et chacun représente un groupe méthyle ; et
R² et R³ ensemble avec l'atome C¹ auquel ils sont attachés forment un noyau carbocyclique mono- ou bicyclique saturé ou partiellement insaturé en C4-C14, et
R^{2,} et R^{3,} ensemble avec l'atome de carbone (C¹') auquel ils sont attachés forment un noyau carbocyclique mono- ou bicyclique saturé ou partiellement insaturé en C4-C14, en sorte que le système cyclique formé par R² et R³ ensemble avec C¹ soit de préférence identique à un système cyclique formé par R^{2,} et R^{3,} ensemble avec C¹' ; ou
R¹ et R^{1,} sont identiques et représentent un groupe aryle monocyclique non substitué en C5-C7 ;
R² et R^{2,} sont identiques et sont un groupe méthyle ; et
R³ et R^{3,} sont identiques et sont un groupe alkyle à chaîne droite en C6-C30.

2. Composition selon l'une quelconque des revendications précédentes, dans laquelle R² et R³ ensemble avec l'atome de carbone (C¹) auquel ils sont attachés et/ou R^{2,} et R^{3,} ensemble avec l'atome de carbone (C¹') auquel ils sont attachés forment un noyau carbocyclique monocyclique non substitué saturé en C5-C8.

3. Composition selon l'une quelconque des revendications précédentes, dans laquelle le système cyclique formé par R^{2,} et R^{3,} ensemble avec l'atome de carbone (C¹') auquel ils sont attachés est identique au système cyclique formé par R² et R³ ensemble avec l'atome de carbone (C¹) auquel ils sont attachés.

4. Composition selon la revendication 1, dans laquelle l'agent générateur de radicaux libres est un composé choisi parmi l'un quelconque des suivants :
- le peroxyde de di(1-méthyl-1-phénylundécyle),
- le peroxyde de di(1-méthyl-1-phénylheptyle),
- le peroxyde de di(1-méthylcyclohexyle) ou
- le peroxyde de di(1-méthylcyclopentyle).

5. Procédé de réticulation d'une composition polymère par réaction radicalaire en utilisant un agent générateur de radicaux libres, **caractérisé en ce qu'**une réaction radicalaire est initiée, par exemple, par chauffage dans une composition polymère comprenant un agent générateur de radicaux libres qui est un composé selon les revendications 1 à 4 pour effectuer de la sorte une réticulation.

6. Câble réticulable qui comprend un conducteur qui est entouré d'une ou de plusieurs couches comprenant une composition polymère, **caractérisé en ce que** la ou les couches comprend ou comprennent une composition polymère telle que définie dans l'une quelconque des revendications 1 à 4.

7. Câble réticulable selon la revendication 6, qui comprend au moins une couche parmi une couche isolante, une couche semi-conductrice ou une couche de gainage qui comprend une composition polymère telle que définie dans l'une quelconque des revendications 1 à 4 ; ou
- un câble de faible tension comprenant un conducteur entouré par une couche isolante et facultativement une couche de gainage, ladite couche isolante comprenant une composition polymère telle que définie dans l'une quelconque des revendications 1 à 4 ; ou
- un câble d'alimentation comprenant un conducteur électrique entouré par une ou plusieurs couches comprenant au moins une couche semi-conductrice, une couche isolante et une couche semi-conductrice extérieure, dans cet ordre, et entourée(s) facultativement par une couche de gainage, au moins une desdites couches comprenant, de préférence au moins une couche semi-conductrice interne et une couche isolante, une composition polymère telle que définie dans l'une quelconque des revendications 1 à 4.

8. Procédé de réticulation d'un câble par le biais d'une réaction radicalaire, comprenant les étapes consistant à :
- appliquer une ou plusieurs couches comprenant une composition polymère selon les revendications 1 à 4 sur un conducteur et
- réticuler par le biais d'une réaction radicalaire ladite au moins une couche comprenant ledit ou lesdits agents générateurs de radicaux libres,
- soumettre le câble ainsi obtenu à une autre étape de refroidissement, ledit câble réticulé étant refroidi dans des conditions sous pression,
et facultativement après ladite étape de refroidissement, le câble réticulé et refroidi est soumis à une ou plusieurs autres étapes choisies parmi :
- une étape de refroidissement sans pression, dans laquelle le câble réticulé et refroidi est davantage refroidi dans un milieu de refroidissement,
- une étape de récupération, dans laquelle le câble réticulé est recueilli après l'étape de refroidissement, de préférence enroulé en une bobine de câble,
- une étape de dégazage, dans laquelle la teneur en produit(s) de décomposition volatil(s) est réduite ou éliminée, facultativement à température ambiante ou à une température élevée, dudit câble réticulé obtenu à partir de ladite étape de refroidissement et facultativement de l'étape de récupération, et/ou
- une étape de finition dans laquelle le câble réticulé obtenu est fini d'une manière conventionnelle pour une autre utilisation ;
**caractérisé en ce que** ladite réticulation est réalisée en utilisant un procédé tel que défini selon la revendication 5.

9. Composé qui est un peroxyde organique de formule (III) : 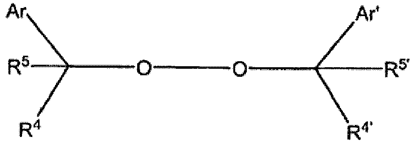
dans lequel Ar et Ar' représentent indépendamment un groupe phényle, benzyle ou naphtyle,
R⁴ et R^{4,} sont chacun un groupe méthyle ; et
R⁵ et R^{5,} représentent chacun indépendamment un groupe alkyle à chaîne droite ayant des atomes de carbone en C6-C30, de préférence en C6-C20, mieux encore en C6-C12 ; ou
le composé de peroxyde de di(1-méthylcyclohexyle).

10. Composé selon la revendication 9, qui est choisi parmi les suivants :
- le peroxyde de di(1-méthyl-1-phénylundécyle) ou
- le peroxyde de di(1-méthyl-1-phénylheptyle).
